Europäisches Patentamt

European Patent Office

Office européen des brevets

(11) Veröffentlichungsnummer: **0 400 638 B1**

## EUROPÄISCHE PATENTSCHRIFT

(45) Veröffentlichungstag der Patentschrift: **05.10.94**

(51) Int. Cl.5: **C12P 13/04**, C12P 7/40

(21) Anmeldenummer: **90110345.7**

(22) Anmeldetag: **31.05.90**

(54) **Verfahren zur Herstellung von D-alpha-Aminocarbonsäuren.**

(30) Priorität: **02.06.89 DE 3918057**

(43) Veröffentlichungstag der Anmeldung:
**05.12.90 Patentblatt 90/49**

(45) Bekanntmachung des Hinweises auf die
Patenterteilung:
**05.10.94 Patentblatt 94/40**

(84) Benannte Vertragsstaaten:
**AT BE CH DE DK ES FR GB IT LI LU NL SE**

(56) Entgegenhaltungen:

BIOTECHNOLOGY & BIOENGINEERING, Band
22, Nr. 9, September 1981, New York, NY
(US); R. OLIVIERI et al., Seiten
2173-2183&NUM;

(73) Patentinhaber: **DEGUSSA AG**
**Weissfrauenstrasse 9**
**D-60311 Frankfurt (DE)**

Patentinhaber: **RECORDATI S.A. CHEMICAL**
**and PHARMACEUTICAL COMPANY**
**Corso S. Gottardo 54**
**CH-6830 Chiasso (CH)**

(72) Erfinder: **Makryaleas, Kyriakos, Dr.**
**Dipl.-Chem.**
**Am Rain 2**
**D-6463 Freigericht 1 (DE)**
Erfinder: **Drauz, Karlheinz, Dr. Dipl.-Chem.**
**Zur Marienruhe 13**
**D-6463 Freigericht 1 (DE)**

## Beschreibung

Die Erfindung betrifft ein Verfahren zur Herstellung von D-$\alpha$-Aminocarbonsäuren durch Biotransformation von in 5-Stellung monosubstituierten Hydantoinen in einem wäßrigen Medium mit einem pH-Wert von mindestens 6,5 in Gegenwart von Zellen des Mikroorganismus Agrobacterium radiobacter.

D-$\alpha$-Aminocarbonsäuren stellen wertvolle Zwischenprodukte für die Herstellung von pharmazeutischen Wirkstoffen (z.B. D-Phenylglycin und D-4-Hydroxyphenylglycin für die Synthese von Penicillinen und Cephalosporinen) oder Pestiziden [z.B. D-Valin für die Synthese des Insektizids Fluvalinat) dar. D-Alanin findet bei der Herstellung des diätetischen Süßstoffes Alitam® Anwendung. $\omega$-Ureidoalkyl-D-$\alpha$-aminocarbonsäuren und $\omega$-Ureidoheteroalkyl-D-$\alpha$-aminocarbonsäuren sind Analoga des D-Citrullins. D-Citrullin und D-Homocitrullin finden Verwendung in potenten LH-RH-Antagonisten und stellen somit wertvolle Synthesebausteine dar.

Aus Biotechnology and Bioengineering, Vol. XXIII, Pp. 2173-2183 (1981) ist die Herstellung verschiedener D-$\alpha$-Aminocarbonsäuren durch Biotransformation von in 5-Stellung entsprechend substituierten Hydantoinen in einem wäßrigen Medium mit einem pH-Wert von mindestens 6,5 in Gegenwart von Zellen des Mikroorganismus Agrobacterium radiobacter grundsätzlich bekannt. Die Biotransformation wird unter Atmosphärendruck vorgenommen. Die Gesamtausbeute beträgt im Falle des D-4-Hydroxyphenylglycins lediglich 60 %.

Das erfindungsgemäße Verfahren ist nun dadurch gekennzeichnet. daß man die Biotransformation in einem geschlossenen Reaktor vornimmt, in diesem Reaktor zu Reaktionsbeginn einen Überdruck zwischen 1 bar und 30 bar einstellt und diesen Überdruck für einen Zeitraum von mindestens 16 Stunden aufrechterhält.

Überraschenderweise ergibt sich bei Anwendung eines Überdruckes bei Reaktionsbeginn eine wesentliche Erhöhung der Raum-Zeit-Ausbeute. Wird nach einigen Stunden der Reaktor entspannt und die Biotransformation bei Atmosphärendruck weitergeführt, so wird die Raum-Zeit-Ausbeute noch weiter verbessert.

Besonders vorteilhaft ist es, den Überdruck für einen Zeitraum von 16 bis 30 Stunden, vorzugsweise von 20 bis 25 Stunden, aufrechtzuerhalten, dann zu entspannen und die Biotransformation für einen weiteren Zeitraum von 18 bis 32 Stunden, vorzugsweise von 23 bis 28 Stunden, bei Atmosphärendruck vorzunehmen.

Der Überdruck wird zweckmäßigerweise durch Aufpressen eines Inertgases, vorzugsweise Stickstoff, erzeugt. Er wird für einen Zeitraum von 16 bis 30 Stunden, vorzugsweise von 20 bis 25 Stunden, aufrechterhalten. Bevorzugt wird ein Druck zwischen 2 und 10 bar.

Mit Hilfe des erfindungsgemäßen Verfahrens kann eine Vielzahl von in 5-Stellung monosubstituierten Hydantoinen in die entsprechenden D-$\alpha$-Aminocarbonsäuren umgewandelt werden. Als Substituenten in 5-Stellung kommen beispielsweise infrage geradkettige, verzweigte oder cyclische Alkylreste mit 1 bis 12 Kohlenstoffatomen, wie Methyl-, Ethyl-, n-Propyl-, Isopropyl-, Isobutyl-, 1-Methylpropyl-, tert. Butyl- , Cyclopentyl- oder Cyclohexylreste; geradkettige, verzweigte oder cyclische ungesättigte Kohlenwasserstoffreste, wie 2-Propenyl-, 2-Butenyl-, 1-Cyclohexenyl oder 1,4-Cyclohexadienylreste; Kohlenwasserstoffreste der genannten Art, die ein- oder mehrfach durch Hydroxyl-, Carboxyl-, Sulfhydryl-, Alkylmercapto-, Amino-, Alkylamino-, Alkoxy-, Carbamoyl-, Guanidino-, Ureido-, Ureidoalkyl-oxyalkyl-, Ureidoalkyl-mercaptoalkyl-, Ureidoalkyl-aminoalkyl-, Sulfoxyl-, Nitro-, Halogen-, Acyl-, Aminosulfenyl-, Arylmercapto-, 4-Imidazolyl-oder 4-Thienylgruppen substituiert sind; aromatische Kohlenwasserstoffreste, wie Phenyl- oder Naphtylreste, die gegebenenfalls ein-oder mehrfach durch Alkyl-, Alkenyl-, cyclische Alkyl-oder Alkenyl-, Hydroxyl-, Alkoxyl-, Halogen-, Benzyloxy-, Benzyloxymethyloxy-, Methoxymethyloxy-, Acyloxy-, Acyl-, Aryloxy-, Amino-, Acylamino-, Alkylamino-, Nitro-, Carboxyl- und Carbamoylgruppen substituiert sind.; heteroaromatische Reste, wie 2-Thienyl-, 5-Thiazolyl-, 4-Imidazolyl- oder 2-Furylreste, die gegebenenfalls ein- oder mehrfach durch die vorstehend bereits genannten Gruppen substituiert sind, Aralkyl- oder Heteroarylalkylreste, die gegebenenfalls ein- oder mehrfach durch die vorstehend bereits genannten Gruppen substituiert sind.

Die in 5-Stellung monosubstituierten Hydantoine können beispielsweise durch Strecker-Synthese aus den entsprechenden Aldehyden, Balusäure und Ammoniak/Kohlendioxid oder Ammoniumcarbonat hergestellt werden. Wenn die entsprechenden Aldehyde nicht verfügbar sind, können die Hydantoine alternativ auch aus den entsprechenden $\alpha$-Aminocarbonsäuren in an sich bekannter Weise durch Umsetzung mit Kaliumcyanat und anschließendes Behandeln der zunächst gebildeten N-Carbamoyl-$\alpha$-aminocarbonsäuren mit einer Säure hergestellt werden. Dabei können die $\alpha$-Aminocarbonsäuren Sowohl in der L- als auch in der D,L-Form oder als Teilracemate eingesetzt werden.

Das erfindungsgemäße Verfahren wird im allgemeinen in einem wäßrigen Medium mit einem pH-Wert zwischen 6,5 und 11,0 , vorzugsweise zwischen 7,0 und 9,0 , durchgeführt. Die zweckmäßige Temperatur

EP 0 400 638 B1

liegt zwischen 20° C und 60° C, vorzugsweise zwischen 35° C und 45° C.

Die Einsatzkonzentration der in 5-Stellung monosubstituierten Hydantoine soll im allgemeinen 5 bis 30 Gewichtsprozent betragen. Aufgrund der häufig schlechten Löslichkeit der Hydantoine liegen diese zunächst oft in suspendierter Form vor. Dies ist jedoch kein Hindernis für die Biotransformation, weil ständig das verbrauchte Substrat durch neu gelöstes ersetzt wird.

Infolge der D-Stereospezifität der in dem Mikroorganismus Agrobacterium radiobacter wirksamen Enzyme werden grundsätzlich nur die D-Enantiomeren der Hydantoine umgesetzt. Da aber unter den Reaktionsbedingungen die Hydantoine spontan racemisieren, werden auch die L-Enantiomeren vollständig in die entsprechenden D-α-Aminocarbonsäuren umgewandelt.

Die einzusetzende Menge an Biokatalysator (Zellen) hängt von der Affinität des jeweiligen Substrates zu den wirksamen Enzymen ab. Im allgemeinen ist eine Menge zweckmäßig, die zum eingesetzten Hydantoin im Gewichtsverhältnis zwischen 1:4 und 1:40 steht.

Die nach dem erfindungsgemäßen Verfahren hergestellten D-α-Aminocarbonsäuren können auf einfache Weise isoliert werden, z.B. durch Anwendung von Ionenaustauschern oder durch Ausfällung beim jeweiligen isoelektrischen Punkt.

Die folgenden Beispiele und Vergleichsversuche sollen das erfindungsgemäße Verfahren näher erläutern. Die Bestimmung des Umsatzes erfolgt jeweils über die quantitative Bestimmung der entstandenen D-α-Aminocarbonsäure mittels Hochleistungsflüssigkeitschromatographie.

Beispiel 1:

Eine Suspension von 60 g D,L-Isopropylhydantoin in 716,7 g $H_2O$ mit einem pH-Wert von 8,4 wurde in einen 1 ℓ-Büchi-Laborautoklaven überführt und bei 40° C etwa 10 Minuten lang mit $N_2$ begast. Dann wurden 83,3 g Biomasse-Suspension in den Reaktor zugegeben. Die Suspension wurde schwach gerührt und weitere 10 Minuten lang mit $N_2$ begast.

Danach wurde der Reaktor verschlossen und mit $N_2$ ein Überdruck von 1,0 bar eingestellt. Die Reaktion verlief bei 40° C unter schwachem Rühren. Nach 21 Stunden wurde der Reaktor entspannt und nach weiteren 27 Stunden bei 40° C war die Reaktion beendet. Der Endumsatz betrug 95 % der Theorie.

Nach Abtrennung der Biomasse über eine Ultrafiltrationsmembran wurde die Produktlösung auf pH 6,0 eingestellt, im Rotationsverdampfer eingeengt und unter Kühlung mit Methanol versetzt. Mit 43,0 g isoliertem D-Valin betrug die Isolierausbeute 87 %, bezogen auf das eingesetzte D,L-Isopropylhydantoin. Das isolierte Produkt zeigte folgende analytische Werte:

| Gehalt (HPLC) | ~ 100 % (nur 1 Peak) |
|---|---|
| Spezifische Drehung | $[\alpha]_D^{20}$ = -28,0° (c = 8 in 6N HCl) |
| D,L-Verteilung | D-Val = 99,83 %; L-Val 0,17 %. |

Vergleichsversuch 1:

Das Beispiel 1 wurde wiederholt mit dem Unterschied, daß in dem Reaktor kein Überdruck eingestellt wurde. Nach 48 Stunden betrug der Umsatz nur 60 % der Theorie.

Beispiel 2:

Das Beispiel 1 wurde wiederholt mit dem Unterschied, daß der Reaktor nach 21 Stunden nicht entspannt, sondern der Überdruck 48 Stunden lang aufrechterhalten wurde. Der Endumsatz betrug 85 % der Theorie.

Beispiel 3:

Es wurde verfahren wie im Beispiel 1, jedoch wurden 60 g D,L-Methylhydantoin eingesetzt und es wurde ein Überdruck von 3,8 bar eingestellt. Der Endumsatz betrug 93 % der Theorie und die Isolierausbeute an D-Alanin 84 %, bezogen auf das eingesetzte D,L-Methylhydantoin. Das isolierte Produkt zeigte folgende analytische Werte:

3

| Gehalt (HPLC) | ~ 100 % (nur 1 Peak) |
|---|---|
| Spezifische Drehung | $[\alpha]_D^{20} = -14{,}2°$ (c = 10 in 6N HCl). |

Vergleichsversuch 2:

Das Beispiel 3 wurde wiederholt mit dem Unterschied, daß in dem Reaktor kein Überdruck eingestellt wurde. Nach 48 Stunden betrug der Umsatz nur 56 % der Theorie.

Beispiel 4:

Es wurde verfahren wie im Beispiel 1, jedoch wurden 60 g D,L-Phenylhydantoin und 41,6 g Biomasse-Suspension eingesetzt und es wurde ein Überdruck von 6,0 bar eingestellt. Nach 48 Stunden betrug der Endumsatz über 98 % der Theorie. Das isolierte D-Phenylglycin zeigte folgende analytische Werte:

| Gehalt (HPLC) | ~ 100 % (nur 1 Peak) |
|---|---|
| Spezifische Drehung | $[\alpha]_D^{20} = -155{,}3°$ (c = 1 in 1N HCl). |

Vergleichsversuch 3:

Das Beispiel 4 wurde wiederholt mit dem Unterschied, daß in dem Reaktor kein Überdruck eingestellt wurde. Nach 48 Stunden betrug der Umsatz nur 59 % der Theorie.

Beispiel 5:

Es wurde verfahren wie im Beispiel 1, jedoch wurden 60 g 5-(4′ -Hydroxyphenyl)-hydantoin eingesetzt und es wurde ein Überdruck von 6,0 bar eingestellt. Nach 20 Stunden unter Überdruck betrug der Endumsatz 100 % der Theorie. Das isolierte D-(4-Hydroxyphenyl)-glycin zeigte folgende analytische Werte:

| Gehalt (HPLC) | ~ 100 % (nur 1 Peak) |
|---|---|
| Spezifische Drehung | $[\alpha]^{20}_D = -158{,}4°$ (c = 1 in 1 N HCl). |

Vergleichsversuch 4:

Das Beispiel 5 wurde wiederholt mit dem Unterschied, daß in dem Reaktor kein Überdruck eingestellt wurde. Nach 20 Stunden betrug der Umsatz nur 63 % der Theorie.

Beispiel 6:

Es wurde verfahren wie im Beispiel 1, jedoch wurden 60 g D,L-Pyridylmethylhydantoin eingesetzt und es wurde ein Überdruck von 6,0 bar eingestellt. Nach 48 Stunden betrug der Endumsatz 100 % der Theorie. Die Isolierausbeute an 3-(2′-Pyridyl)-D-alanin lag bei 93 % der Theorie. Das isolierte Produkt zeigte folgende analytische Werte:

| Gehalt (HPLC) | ~ 100 % (nur 1 Peak) |
|---|---|
| Spezifische Drehung | $[\alpha]_D^{20} = -26{,}21°$ (c = 1 in 2N HCl) |
| D,L-Verteilung | D = 100 %; L = 0 %. |

EP 0 400 638 B1

Beispiel 7:

Es wurde verfahren wie im Beispiel 1, jedoch wurden 60 g D,L-Naphthylmethylhydantoin eingesetzt und es wurde ein Überdruck von 6,0 bar eingestellt. Nach 48 Stunden betrug der Endumsatz 92 % der Theorie. Das isolierte Produkt zeigte folgende analytische Werte:

| Gehalt (HPLC) | ~ 100 % (nur 1 Peak) |
|---|---|
| Spezifische Drehung | $[\alpha]_D^{20} = +2{,}6°$ (c = 2 in 2N NaOH) |
| D,L-Verteilung | D = 99,4 %, L = 0,6 %. |

Beispiel 8:

Es wurde verfahren wie im Beispiel 1, jedoch wurden 60 g L-Citrullinhydantoin eingesetzt und es wurde ein Überdruck von 6,0 bar eingestellt. Nach 48 Stunden betrug der Endumsatz 100 % der Theorie und die Isolierausbeute an D-Citrullin 92 %, bezogen auf das eingesetzte Hydantoin. Das isolierte Produkt zeigte folgende analytische Werte:

| Gehalt (HPLC) | ~ 100 % (nur 1 Peak) |
|---|---|
| Spezifische Drehung | $[\alpha]_D^{20} = +24{,}0°$ (c = 2 in 1N HCl). |

Beispiel 9:

Es wurde verfahren wie im Beispiel 1, jedoch wurden 60 g D,L-5-Methylmercaptoethylhydantoin eingesetzt und es wurde ein Überdruck von 1,6 bar eingestellt. Nach 48 Stunden betrug der Endumsatz 94 % der Theorie. Das isolierte Produkt zeigte folgende analytische Werte:

| Gehalt (HPLC) | ~ 100 % (nur 1 Peak) |
|---|---|
| Spezifische Drehung | $[\alpha]^{20}_D = -23{,}9°$ (c = 5 in 5N HCl). |

Beispiel 10:

Es wurde verfahren wie im Beispiel 1, jedoch wurden 60 g L-5-(1'-Hydroxyethyl)-hydantoin (2 S, 3 R) eingesetzt und ein Überdruck von 3,8 bar eingestellt. Nach 48 Stunden betrug der Endumsatz 90 % der Theorie. Das isolierte D-Allothreonin (2 R, 3 R) zeigte folgende analytische Werte:

| Gehalt (HPLC) | ~ 100 % (nur 1 Peak) |
|---|---|
| Spezifische Drehung | $[\alpha]^{20}_D = -32{,}5°$ (c = 1 in 1 N HCl). |

Beispiel 11:

Es wurde verfahren wie im Beispiel 1, jedoch wurden 60 g D,L-5-(3'-Indolylmethyl)-hydantoin eingesetzt und es wurde ein Überdruck von 2,5 bar eingestellt. Nach 48 Stunden betrug der Endumsatz 87 % der Theorie. Das isolierte D-Tryptophan zeigte folgende analytische Werte:

| Gehalt (HPLC) | ~ 100 % (nur 1 Peak) |
|---|---|
| Spezifische Drehung | $[\alpha]^{20}_D = +32{,}1°$ (c = 1 in $H_2O$). |

5

EP 0 400 638 B1

Beispiel 12:

Es wurde verfahren wie im Beispiel 1, jedoch wurden 60 g L-5-(4'-Hydroxybenzyl)-hydantoin eingesetzt und es wurde ein Überdruck von 1,8 bar eingestellt. Nach 48 Stunden betrug der Endumsatz 89 % der Theorie. Das isolierte D-Tyrosin zeigte folgende analytische Werte:

| Gehalt (HPLC) | ~ 100 % (nur 1 Peak) |
|---|---|
| Spezifische Drehung | $[\alpha]^{20}_D$ = + 11,4° (c = 4 in 1 N HCl). |

Beispiel 13:

Es wurde verfahren wie im Beispiel 1, jedoch wurden 60 g L-5-(4'-Imidazoylmethyl)-hydantoin eingesetzt und es wurde ein Überdruck von 1,5 bar eingestellt. Nach 48 Stunden betrug der Endumsatz 86 % der Theorie. Das isolierte D-Histidin zeigte folgende analytische Werte:

| Gehalt (HPLC) | ~ 100 % (nur 1 Peak) |
|---|---|
| Spezifische Drehung | $[\alpha]^{20}_D$ = + 39,6° (c = 5 in $H_2O$). |

Beispiel 14:

Es wurde verfahren wie im Beispiel 1, jedoch wurden 60g 5-(2'-Ureidoethyl)-D,L-hydantoin eingesetzt und es wurde ein Überdruck von 3 bar eingestellt. Nach 48 Stunden betrug der Endumsatz 95 % und die Isolierausbeute nach Reinigung an einem stark sauren Ionenaustauscher 80 % der Theorie. Das isolierte 3-(Ureidomethyl)-D-alanin (D-Norcitrullin) zeigte folgende analytische Werte:

| Gehalt (HPLC) | ~ 100 % (nur ein Peak) |
|---|---|
| Schmelzpunkt | 200-201°C |
| Spezifische Drehung | $[\alpha]^{20}_D$ = -22,4° (c = 1 in 0,5N HCl). |

Beispiel 15:

Es wurde verfahren wie im Beispiel 1, jedoch wurden 60g 5-(Ureidomethyl)-D, L-hydantoin eingesetzt und es wurde ein Überdruck von 3 bar eingestellt. Nach 48 Stunden betrug der Endumsatz 90 % und die Isolierausbeute nach Reinigung an einem stark sauren Ionenaustauscher 80 % der Theorie. Das isolierte 3-Ureido-D-alanin zeigte folgende analytische Werte:

| Gehalt (HPLC) | ~ 100 % (nur ein Peak) |
|---|---|
| Schmelzpunkt | 203-203,5°C |
| Spezifische Drehung | $[\alpha]^{20}_D$ = + 66,8° (c = 1 in $H_2O$). |

Beispiel 16:

Es wurde verfahren wie im Beispiel 1, jedoch wurden 60g 5-(2'-Ureidoethyl-thiomethyl)-L-hydantoin Hydrochlorid eingesetzt und es wurde ein Überdruck von 3,2 bar eingestellt. Nach 48 Stunden betrug der Endumsatz 100 % und die Isolierausbeute nach Reinigung an einem stark sauren Ionenaustauscher 85 % der Theorie. Das isolierte S-(2'-Ureidoethyl)-D-cystein zeigte folgende analytische Werte:

6

| Gehalt (HPLC) | ~ 100 % (nur ein Peak) |
| Schmelzpunkt | 192,5-193,5°C (Zersetzung) |
| Spezifische Drehung | $[\alpha]_D^{20}$ = + 15,3° (c = 1 in $H_2O$). |

Beispiel 17:

Es wurde verfahren wie im Beispiel 1, jedoch wurden 60g 5-(2'-Ureidoethyl-oxymethyl)-D, L-hydantoin eingesetzt und es wurde ein Überdruck von 3,2 bar eingestellt. Hach 48 Stunden betrug der Endumsatz 100 %. Das isolierte O-(2'-Ureidoethyl)-D-serin zeigte folgende analytische Werte:

| Gehalt (HPLC) | ~ 100 % (nur ein Peak |
| NMR ($D_2O$) | 3,86-4,0 (m, 3H) ; 3,52-3,7 (m, 2H); 3,22-3,38 (m, 2H)ppm. |
| $R_f$-Wert-DC | 0,27 (Ethanol/25 %ige $NH_3$-Lösung = 8:2). |

Beispiel 18:

Es wurde verfahren wie im Beispiel 1, jedoch wurden 60g 5-(2'-Ureidoethyl-thioethyl)-D, L-hydantoin Hydrochlorid eingesetzt und es wurde ein Überdruck von 3,2 bar eingestellt. Nach 48 Stunden betrug der Endumsatz 100 % und die Isolierausbeute nach Reinigung an einem stark sauren Ionenaustauscher 85 % der Theorie. Das isolierte S-(2'-Ureidoethyl)-D-homocystein zeigte folgende analytische Werte:

| Gehalt (HPLC) | ~ 100 % (nur ein Peak) |
| Schmelzpunkt | 208,5-209°C (Zersetzung) |
| Spezifische Drehung | $[\alpha]_D^{20}$ = + 6,75° (c = 1 in $H_2O$). |

Beispiel 19:

Es wurde verfahren wie im Beispiel 1, jedoch wurden 60g 5-(2'-Ureidoethyl-thioisopropyl)-D, L-hydantoin eingesetzt und es wurde ein Überdruck von 3,2 bar eingestellt. Nach 48 Stunden betrug der Endumsatz 80 % und die Isolierausbeute an S-(2'-Ureidoethyl)-D-penicillaminnach Reinigung an einem stark sauren Ionenaustauscher 65 % der Theorie.

Beispiel 20:

Es wurde verfahren wie im Beispiel 1, jedoch wurden 60g 5-(2'-Thienyl)-D,L-hydantoin eingesetzt und es wurde ein Überdruck von 1,6 bar eingestellt. Nach 19 Stunden unter Überdruck betrug der Endumsatz 100 % der Theorie. Das isolierte 2-Thienyl-D-glycin zeigte folgende analytische Werte:

| Gehalt (HPCL) | ~ 100 % (nur ein Peak) |
| Schmelzpunkt | 206-209°C |
| Spezifische Drehung | $[\alpha]_D^{20}$ = -108,5° (c = 1 in 1N HCl). |

Beispiel 21:

Es wurde verfahren wie im Beispiel 1, jedoch wurden 60g 5-(1'-Mercapto-1'-methyl-ethyl)-D,L-hydantoin eingesetzt und es wurde ein Überdruck von 3,5 bar eingestellt. Nach 48 Stunden betrug der Endumsatz 65 % der Theorie. Das isolierte D-Penicillamin zeigte folgende analytische Werte:

7

| Gehalt (HPLC) | ~ 100 % (nur ein Peak) |
|---|---|
| Spezifische Drehung | $[\alpha]_D^{25}$ = -63,0° (c = 1 in Pyridin) |
| D,L-Verteilung | D = 99,9 %, L = 0,1 %. |

Vergleichsversuch 5:

Das Beispiel 21 wurde wiederholt mit dem Unterschied, daß in dem Reaktor kein Überdruck eingestellt wurde. Nach 48 Stunden betrug der Umsatz weniger als 10 % der Theorie.

## Patentansprüche

1.  Verfahren zur Herstellung von D-$\alpha$-Aminocarbonsäuren durch Biotransformation von in 5-Stellung monosubstituierten Hydantoinen in einem wäßrigen Medium mit einem pH-Wert von mindestens 6,5 in Gegenwart von Zellen des Mikroorganismus Agrobacterium radiobacter,
    dadurch gekennzeichnet,
    daß man die Biotransformation in einem geschlossenen Reaktor vornimmt, in diesem Reaktor zu Reaktionsbeginn einen Überdruck zwischen 1 bar und 30 bar einstellt und diesen Überdruck für einen Zeitraum von mindestens 16 Stunden aufrechterhält.

2.  Verfahren nach Anspruch 1,
    dadurch gekennzeichnet,
    daß man den Überdruck für einen Zeitraum von 16 bis 30 Stunden aufrechterhält, dann entspannt und die Biotransformation für einen weiteren Zeitraum von 18 bis 32 Stunden bei Atmosphärendruck vornimmt.

3.  Verfahren nach Anspruch 2,
    dadurch gekennzeichnet,
    daß man den Überdruck für einen Zeitraum von 20 bis 25 Stunden aufrechterhält und die Biotransformation für einen weiteren Zeitraum von 23 bis 28 Stunden bei Atmosphärendruck vornimmt.

4.  Verfahren nach einem der Ansprüche 1 bis 3.
    dadurch gekennzeichnet,
    daß man den Überdruck durch Aufpressen eines Inertgases erzeugt.

## Claims

1.  A process for the production of D-$\alpha$-aminocarboxylic acids by biotransformation of hydantoins monosubstituted in the 5-position in an aqueous medium with a pH value of at least 6.5 in the presence of cells of the microorganism Agrobacterium radiobacter, characterized in that the biotransformation is carried out in a closed reactor, an excess pressure of 1 bar to 30 bar is established in the reactor at the beginning of the reaction and is maintained for a period of at least 16 hours.

2.  A process as claimed in claim 1, characterized in that the excess pressure is maintained for a period of 16 to 30 hours and then removed and the biotransformation is continued for another 18 to 32 hours at atmospheric pressure.

3.  A process as claimed in claim 2, characterized in that the excess pressure is maintained for a period of 20 to 25 hours and the biotransformation is continued for another 23 to 28 hours at atmospheric pressure.

4.  A process as claimed in any of claims 1 to 3, characterized in that the excess pressure is established by introducing an inert gas under pressure.

**Revendications**

1. Procédé de préparation d'acides D-α-aminocarboxyliques par biotransformation d'hydantoines mono-substituées en position 5 dans un milieu aqueux ayant une valeur de pH d'au moins 6,5 en présence de cellules du microorganisme Agrobactérium radiobacter, caractérisé en ce que l'on effectue la biotransformation dans un réacteur clos, que l'on ajuste dans ce réacteur jusqu'au démarrage de la réaction une surpression comprise entre 1 bar et 30 bar et que l'on maintient cette surpression pendant un espace de temps d'au moins 16 heures.

2. Procédé selon la revendication 1, caractérisé en ce que l'on maintient la surpression pendant un espace de temps allant de 16 à 30 heures, puis on détend et on effectue la biotransformation pendant un espace de temps supplémentaire de 18 à 32 heures à la pression atmosphérique.

3. Procédé selon la revendication 2, caractérisé en ce que l'on maintient la surpression pendant un espace de temps de 20 à 25 heures et que l'on effectue la biotransformation pendant un espace de temps supplémentaire de 23 à 28 heures à la pression atmosphérique.

4. Procédé selon l'une des revendications 1 à 3, caractérisé en ce que l'on obtient la surpression par l'engagement sous pression d'un gaz inerte.